# EUROPÄISCHE PATENTANMELDUNG

(11) **EP 1 566 637 A1**
(43) Veröffentlichungstag der Anmeldung: **24.08.2005**
(21) Anmeldenummer: 05003434.7
(22) Anmeldetag: 17.02.2005
(51) Int. Cl.: G01N 33/52, C12Q 1/54

(54) **Testelement mit Einschicht-Reaktionsfilm**

(30) Priorität: 18.02.2004 DE 102004007983
(71) Anmelder: F. HOFFMANN-LA ROCHE AG, 4070 Basel (CH); Roche Diagnostics GmbH, 68305 Mannheim (DE)
(72) Erfinder: Knappe, Wolfgang-Reinhold, Dr., 67071 Ludwigshafen (DE); Wittmann, Franz, 68766 Hockenheim (DE); Mosoiu, Dan, 67117 Limburgerhof (DE); Horn, Carina, Dr., 68647 Biblis (DE); Hoenes, Joachim, Dr., 64673 Zwingenberg (DE)
(74) Vertreter: Weiss, Wolfgang

(57) **Zusammenfassung**

Die Erfindung betrifft Testelemente und Verfahren zum optischen Nachweis eines Analyten in einer Probe.

## Beschreibung

Die Erfindung betrifft Testelemente und Verfahren zum optischen Nachweis eines Analyten in einer Probe.

Die qualitative oder quantitative Bestimmung von Analyten in Probenflüssigkeiten wird oft unter Verwendung von trägergebundenen Testverfahren durchgeführt. Bei diesen liegen zur Bestimmung des Analyten erforderliche Reagenzien auf oder in Reaktionsschichten eines festen Testelements vor, mit dem die Probe in Kontakt gebracht wird. Die Reaktion zwischen dem in der Probenflüssigkeit enthaltenen Analyten und den Reagenzien führt zu einem nachweisbaren Signal, insbesondere zu einem optisch nachweisbaren Signal, welches visuell oder mit Hilfe eines Geräts ausgewertet werden kann.

Testelemente sind häufig als Streifen ausgebildet, die aus einem zumindest teilweise optisch im wesentlichen transparenten Träger und darauf aufgebrachte Nachweisschichten bestehen. Es sind jedoch auch Testelemente mit anderer Form bekannt, die z.B. als quadratische oder rechteckige Plättchen gestaltet sind. Im Folgenden soll die Bezeichnung "Testelement" beliebige Formen umfassen.

In DE-A-21 18 455 werden diagnostische Testelemente beschrieben, die aus einem Träger und mindestens einer die Nachweisreagenzien enthaltenden Nachweisschicht aufgebaut sind, deren nicht auf dem Träger liegende Oberfläche mit einer Deckschicht versehen ist. Die Deckschicht kann aus einem feinmaschigen Netzwerk in Form eines Gewebes, Gewirkes oder Vlieses bestehen. Kunststoffgewebe werden als bevorzugte Netzwerke angegeben, um eine schnelle Benetzung der Nachweisschicht mit Probenflüssigkeit zu erreichen und störende Chromatographieeffekte zu vermeiden. Zum Nachweis eines Analyten wird das Testelement in die Probenflüssigkeit eingetaucht. Die Nachweisschicht kommt so mit einem sehr großen Überschuss an Flüssigkeit in Kontakt, der nicht vom Testelement aufgenommen werden kann. Je nach Dauer des Kontakts der Nachweisschicht mit der Probenflüssigkeit können Signale mit unterschiedlicher Stärke beobachtet werden, so dass eine quantitative Auswertung nur eingeschränkt möglich ist.

Andererseits ist ein für ein Testelement zu geringes Probenvolumen eine häufige Ursache für falsche Messwerte beim Diabetes Monitoring, d.h. bei der regelmäßigen Kontrolle des Blutes Diabeteskranker auf den Gehalt an Glucose. Testelemente mit möglichst geringem Bedarf an Probenvolumen sind deshalb das Ziel derzeitiger Entwicklungen. Solche Testträger müssen jedoch nicht nur mit sehr kleinen Probenvolumina von etwa 3 µl korrekte Messwerte ergeben, sondern sie müssen auch bei größeren Probenvolumina von etwa 15-20 µl zuverlässig arbeiten und eine Rückhaltung der Probenflüssigkeit ermöglichen.

Derzeit verwendete Teststreifen zur optischen Bestimmung von Analyten enthalten üblicherweise mindestens zwei Reaktions- bzw. Nachweisschichten, die auf einem Träger übereinander aufgebracht sind. Die erste Schicht ist dabei die Reagenzschicht, die zweite Schicht eine Sperrschicht und eine - insbesondere für einen reflexionsphotometrischen Nachweis - benötige Reflexionsschicht. In der Reagenzschicht, in der die Nachweisreaktion stattfindet, sind üblicherweise neben den Reagenzien nur Füllstoffe enthalten, während die Sperr- bzw. Reflexionsschicht, die das Messlicht reflektieren soll, Pigmente enthalten soll. Durch das Aufbringen dieser zwei Schichten ergeben sich Trockenschichtdicken von ca. 20-40 µm. Beispiele für einen solchen Zwei-Schichtenaufbau sind in DE-A-30 42 857, EP-A-0 821 233 und EP-A-0 995 994 beschrieben. Auch verschiedene kommerzielle Testsysteme verwenden einen solchen Mehrschichtenaufbau, wie etwa das System Drichem 1000 der Firma Fuji (Sonntag, Trockenchemie, Seiten 18 ff., Thieme 1988).

Beim Nachweissystem Ektachem der Firma Kodak erfolgt die Bestimmung von Analyten aus einer Probenflüssigkeit in dünnen Schichten aus Gelatine oder Agarose, in denen die Nachweisreagenzien eingebettet sind (Sonntag, Trockenchemie, Seiten 23 ff., Thieme 1988).

Den oben genannten Verfahren des Standes der Technik gemeinsam sind relativ lange Messzeiten, die im Bereich von >15 s liegen. Durch Verminderung der Schichtdicke der Sperr- bzw. Reaktionsschicht auf ca. 10 µm (trocken) konnten diese Messzeiten auf ca. 5 s verkürzt werden (EP-A-0 995 994). Eine weitere Verminderung der Reaktionszeit durch Verringerung der Schichtdicke konnte jedoch nicht erreicht werden. Ein anderer Nachteil bei Testelementen mit einem Mehr-Schichten-Aufbau besteht darin, dass der Beschichtungs- und Trocknungsprozess zum Aufbringen der Schichten auf den Träger mehrfach durchzuführen ist, was einen erheblichen Arbeitsaufwand darstellt.

Bei dem Ektachem-Nachweisverfahren wiederum werden sehr lange Vorinkubationszeiten, d.h. Zeiten zwischen Probenauftrag und Messung, von etwa 5 min bei 37 °C benötigt.

Eine der Erfindung zugrunde liegende Aufgabe bestand somit darin, ein Testelement bereitzustellen, welches eine zuverlässige quantitative Bestimmung von Analyten bei kurzer Reaktionsdauer ermöglicht und welches einfach hergestellt werden kann.

Diese Aufgabe wird gelöst durch ein Testelement zum optischen Nachweis eines Analyten, umfassend:
(a) einen zumindest teilweise optisch im wesentlichen transparenten Träger und
(b) einen Einschicht-Reaktionsfilm, enthaltend ein Testreagenz zur Bestimmung des Analyten mit einer Dicke ≤ 10 µm (trocken).

Ein weiterer Aspekt der Erfindung betrifft ein Testelement zum optischen Nachweis eines Analyten, umfassend:
(a) einen zumindest teilweise optisch im wesentlichen transparenten Träger und
(b) einen Einschicht-Reaktionsfilm, enthaltend ein Testreagenz zur Bestimmung des Analyten,
dadurch gekennzeichnet, dass es für eine Bestimmung des Analyten innerhalb einer Reaktionsdauer von < 5 s, insbesondere von 1-4 s, vorgesehen ist.

Noch ein Aspekt der Erfindung betrifft ein Testelement zum optischen Nachweis eines Analyten, umfassend:
(a) einen zumindest teilweise optisch im wesentlichen transparenten Träger und
(b) einen Einschicht-Reaktionsfilm, enthaltend ein Testreagenz zur Bestimmung des Analyten,
dadurch gekennzeichnet, dass die Dicke und Porosität des Reaktionsfilms und die Konzentration des Testreagenz derart sind, dass eine Bestimmung des Analyten innerhalb der Reaktionsdauer von < 5 s, insbesondere von 1-4 s, bei einer für eine quantitative Bestimmung ausreichenden Signalintensität erfolgt.

Darüber hinaus betrifft die Erfindung ein Verfahren zum Nachweis eines Analyten in einer Probe, umfassend die Schritte:
(a) Inkontaktbringen der Probe mit einem erfindungsgemäßen Testelement und
(b) quantitative Bestimmung der Analytkonzentration in der Probe.

Es hat sich gezeigt, dass durch Verwendung von Einschicht-Reaktionsfilmen mit geringer Dicke, insbesondere in Kombination mit darauf abgestimmten Partikelgrößen der den Reaktionsfilm bildenden Materialien oder/und der Reagenzkonzentration im Reaktionsfilm, ein Testelement erhalten werden kann, welches einen zuverlässigen quantitativen Nachweis innerhalb einer sehr kurzen Reaktionszeit von insbesondere etwa 1-2 s erlaubt.

Das erfindungsgemäße Testelement ist für optische Nachweismethoden, insbesondere für reflexionsphotometrische oder fluorimetrische Nachweismethoden geeignet. Der optische Nachweis kann im sichtbaren Bereich von 400-800 nm oder/und im UV-Bereich erfolgen. Vorzugsweise besitzt der Einschicht-Reaktionsfilm eine poröse Struktur, insbesondere für reflexionsphotometrische Anwendungen. Das Testreagenz kann gegebenenfalls in einer Polymermatrix vorliegen.

Komponente (a) des erfindungsgemäßen Testelements ist ein zumindest teilweise optisch im wesentlichen transparenter Träger. Dieser Träger ist vorzugsweise ein flüssigkeitsundurchlässiger Träger, beispielsweise eine Kunststofffolie, wie Polycarbonat, Polystyrol, Polyvinylchlorid, Polyester oder Polyamid. Als weiteres Trägermaterial eignet sich beispielsweise Glas. Die Dicke des Trägers ist im Allgemeinen nicht kritisch und liegt vorzugsweise im Bereich von 10 µm bis 1 mm.

Der Träger ist zumindest teilweise, d.h. zumindest in dem zur Bestimmung des Analyten dienenden Bereich (Nachweiszone), optisch im wesentlichen transparent, so dass ein optischer Nachweis des Analyten durch den Träger hindurch erfolgen kann.

Komponente (b) des Testelements ist der Einschicht-Reaktionsfilm, der das Testreagenz zur Bestimmung des Analyten enthält. Das Testreagenz umfasst die zum qualitativen Nachweis oder zur quantitativen Bestimmung des Analyten benötigten Substanzen, zumindest jedoch einen Teil davon, sowie gegebenenfalls Hilfs- oder/und Zusatzstoffe. Der Einschicht-Reaktionsfilm besteht aus einer einzigen Funktionsschicht. Die Dicke des Einschicht-Reaktionsfilms ist vorzugsweise ≤ 10 µm. Vorzugsweise enthält das Testreagenz mindestens ein Enzym und mindestens ein nach enzymatischer Reaktion direkt oder indirekt nachweisbares Enzymsubstrat, beispielsweise ein chromogenes oder ein fluoreszierendes Enzymsubstrat.

Bevorzugte Beispiele für Enzyme sind die zum Glucosenachweis verwendeten Enzyme Glucose-Dehydrogenase, z.B. Glucose-Dehydrogenase in Kombination mit Diaphorase oder PQQ-abhängige Glucose-Dehydrogenase, oder Gluc-DOR (Glucose-Dye-Oxidoreduktase) und Mutanten davon. Die Enzyme können gegebenenfalls in rekombinanter Form eingesetzt werden. Ein solches Testreagenz ist zur Bestimmung von Glucose in Körperflüssigkeiten, insbesondere insbesondere zur Bestimmung von Glucose im Blut, geeignet. Weitere bevorzugte Analyten sind Lactat (Milchsäure), 3-Hydroxybuttersäure (Ketonkörper) oder Harnsäure.

Das Testreagenz bzw. Komponenten davon liegen in trockener Form in bzw. auf dem Einschicht-Reaktionsfilm vor. Das Aufbringen des Testreagenz auf den Reaktionsfilm kann nach bekannten Methoden, beispielsweise durch Imprägnieren, erfolgen. Gegebenenfalls kann das Testelement auch in einer Polymermatrix vorliegen, wobei das Polymer ein natürliches oder synthetisches filmbildendes Polymer, z.B. ein Polyvinylester, Polyvinylacetat, Polyacrylsäure, Polyacrylester, Polyacrylamid, Polymethacrylsäure, Polymethacrylester, Polyvinylamid, Polyamid, Polystyrol oder ein Co- oder Mischpolymer, z.B. von Butadien, Styrol und Maleinsäureester, ist. Das Polymer kann in fertigem Zustand auf den Träger aufgebracht oder durch *in situ* Polymerisation, vorzugsweise in Gegenwart des Testreagenz, auf dem Träger erzeugt werden kann. Weiterhin kann der Reaktionsfilm ein oder mehrere Netzmittel, z.B. zwei Netzmittel wie in EP-A-0 995 994 beschrieben, Pigmente oder/und Quellmittel, z.B. Xanthangummi oder Methylvinylethermaleinsäure-Copolymer, enthalten.

Die Konzentration des Testreagenz im Einschicht-Reaktionsfilm wird günstigerweise derart eingestellt, dass eine im wesentlichen gleichmäßige Reaktion mit dem Analyten über im wesentlichen die gesamte Dicke des Reaktionsfilms erfolgen kann. Entgegen der Annahme eines Fachmanns, dass z.B. eine hohe Konzentration des Testreagenz auch eine hohe Signalintensität verursacht, wurde erfindungsgemäß festgestellt, dass eine optimale Reagenzkonzentration ungleich einer maximalen Reagenzkonzentration ist.

Bei Verwendung einer zu hohen Reagenzkonzentration wird zunächst auf nur einer Seite (auf der die Probe zugegeben wird) eine beschleunigte Umsetzung des Analyten erfolgen. Auf der anderen Seite (d.h der dem Träger zugewandten Seite) findet hingegen nur eine schwächere Umsetzung statt, die - bei einem optischen Nachweis von der unteren Schichtseite her - nur zu schwachen Signalintensitäten führt. Eine zu geringe Reagenzkonzentration führt insgesamt nur zu einer schwachen Umsetzung des Analyten und somit ebenfalls zu einer schwachen Signalintensität. Eine geeignete Reagenzkonzentration für ein gegebenes Testelement kann durch Bestimmung der Signalintensität in Abhängigkeit von der Konzentration des Testreagenz auf einfache Weise ermittelt werden.

In einer bevorzugten Ausführungsform enthält das Testelement somit eine Konzentration des Testreagenz, die durch Optimierung der Intensität eines Messsignals eingestellt ist. Vorzugsweise erfolgt die Einstellung einer optimierten Konzentration des Testreagenz bei einer gegebenen Porosität oder/und Schichtdicke des Testelements.

Ein weiteres wichtiges Merkmal für den Aufbau des Einschicht-Reaktionsfilms ist die Durchlässigkeit für die Probenflüssigkeit. Diese Durchlässigkeit kann durch Einstellung der Porosität bzw. der Partikelgröße der den Reaktionsfilm bildenden Materialien optimiert werden. Vorzugsweise ist der Reaktionsfilm dabei so ausgebildet, dass rote Blutkörperchen auf der oberen Seite des Testelements zurückgehalten werden, während gelöste Probenbestandteile, wie der Analyt, in den Reaktionsfilm eindringen können. Im Testelement reagiert der Analyt mit dem Testreagenz, wobei ein optisch nachweisbares Reaktionsprodukt gebildet wird, z.B. im Beispiel der Glucosebestimmung NADH. Dabei stellt sich ein Diffusionsgleichgewicht zwischen dem oben auf dem Testelement zurückgehalten Blut und dem Inneren des Reaktionsfilms ein, wobei der Analyt, z.B. Glucose, in den Reaktionsfilm hinein diffundiert und das nachweisbare Reaktionsprodukt aus dem Reaktionsfilm hinaus in die Probe zurück diffundiert. Erst bei Einstellung des Diffusionsgleichgewichts kann die Konzentration des Analyten unabhängig von der Zeit vermessen werden. Die Porosität des Analyten ist folglich so zu wählen, dass das Diffusionsgleichgewicht möglichst schnell erreicht wird. Auch hier können optimale Porositäten durch einfache Bestimmungen der Signalintensität ermittelt werden.

In einer weiteren bevorzugten Ausführungsform ist die Porengröße des Einschicht-Reaktionsfilms durch Optimierung der Intensität eines Messsignals eingestellt. Die Optimierung kann beispielsweise unter Bedingungen erfolgen, bei denen eine gegebene Konzentration des Testreagenz oder/und eine gegebene Schichtdicke verwendet werden.

Insgesamt zeigt sich, dass eine Optimierung von Reagenzkonzentration, Porengröße und Schichtdicke entgegen den im Stand der Technik üblichen Annahmen erfolgen kann. Eine Anpassung der Faktoren aneinander ermöglicht eine hinreichende Signalintensität bei einschichtigen Testelementen.

Überraschenderweise kann daher eine hervorragende Messsignalintensität unter Verwendung eines Einschicht-Reaktionsfilm mit einer Dicke von ≤ 10 µm (trocken), vorzugsweise im Bereich von 2-10 µm (trocken) und besonders bevorzugt im Bereich von 5-8 µm (trocken) erreicht werden. Dies führt dazu, dass eine quantitative Bestimmung des Analyten innerhalb einer sehr kurzen Reaktionsdauer von < 5 s, insbesondere von 1-4 s, besonders bevorzugt von 1-2 s, erfolgen kann.

In einer bevorzugten Ausführungsform ist das erfindungsgemäße Testelement zum reflexionsphotometrischen Nachweis eines Analyten vorgesehen. In dieser Ausführungsform enthält der Einschicht-Reaktionsfilm feinkörnige Pigmentpartikel, beispielsweise Pigmentpartikel auf Basis von TiO₂ oder/und ZrO₂. Der mittlere Durchmesser der feinkörnigen Pigmentpartikel, die eine optische Reflexion des Messsignals in Richtung des Detektors und somit eine Erhöhung der Signalintensität bewirken, ist vorzugsweise s 0,5 µm, insbesondere zwischen 0,1 µm und 0,3 µm. Gute Ergebnisse wurden beispielsweise bei einer mittleren Partikelgröße von etwa 0,2 µm erhalten, wie sie typischerweise in kommerziellen Titandioxidpigmenten, z.B. Typ RN 56 oder E 171 der Fa. Kronos, Leverkusen, Deutschland, vorliegt.

Zusätzlich enthält der Einschicht-Reaktionsfilm günstigerweise mittel- oder/und grobkörnige Partikel. Ohne diese Partikel beträgt der Remissionshub für den gesamten Glucosemessbereich 0-600 mg/dl nur etwa 5 %, was bei einer klinisch erforderlichen Präzision von besser 4 % unter Umständen nicht ausreicht. Vorzugsweise handelt es sich bei diesen Partikeln um: (i) mittel- oder/und grobkörnige Pigmentpartikel, beispielsweise Pigmentpartikel auf Basis von TiO₂ oder/und BaSO₄, oder/und (ii) mittel- oder/und grobkörnige Partikel eines nicht reflektierenden Füllmaterials, beispielsweise SiO₂, oder/und eines Silikats oder/und eines wasserunlöslichen Kunststoffs enthält.

Die mittel- oder/und grobkörnigen Pigmentpartikel werden beispielsweise aus TiO₂, BaTiO₃, ZrO₂, ZrSiO₃ oder/und BaSO₄ ausgewählt. Die mittel- oder/und grobkörnigen Partikel eines nicht reflektierenden Füllmaterials werden beispielsweise aus SiO₂ (z.B. Fällungskieselsäure FK 320 der Fa. Degussa), Silikaten (z.B. Transpafill der Fa. Degussa) oder aus unlöslichen organischen Kunststoffen (z.B. quervernetztes Polyvinylpyrrolidon wie Kollidon CL-M der Fa. BASF, Ludwigshafen) ausgewählt. Der mittlere Durchmesser von mittelkörnigen Partikeln liegt vorzugsweise im Bereich zwischen 0,7 und ≤ 4 µm, insbesondere zwischen 1 und 3 µm. Der mittlere Durchmesser von grobkörnigen Partikeln liegt vorzugsweise im Bereich von ≥ 4 µm, insbesondere zwischen 4 und 8 µm.

In einer bevorzugten Ausführungsform enthält das Testelement ein Gemisch aus feinkörnigen Pigmentpartikeln und grobkörnigen Partikeln (i) oder/und (ii) insbesondere in einem Gewichtsverhältnis von 1 (feinkörnige Pigmentpartikel) : 0,2-1 (grobkörnige Partikel).

In einer weiteren bevorzugten Ausführungsform enthält der Einschicht-Reaktionsfilm des Testelements ein Gemisch aus feinkörnigen Pigmentpartikeln und mittelkörnigen Partikeln (i) oder/und (ii), insbesondere in einem Gewichtsverhältnis von 1 (feinkörnige Partikel) : 0,2-1 (mittelkörnige Partikel).

In noch einer weiteren bevorzugten Ausführungsform enthält das Testelement ein Gemisch aus aus feinkörnigen Pigmentpartikeln, grobkörnigen Partikeln (i) oder/und (ii) und mittelkörnigen Partikeln (i) oder/und (ii), insbesondere in einem Gewichtsverhältnis von 1 (feinkörnige Partikel) : 0,1-0,9 (grobkörnige Partikel) : 0,1-0,9 (mittelkörnige Partikel).

Insbesondere bei Verwendung von ZrO₂ als Pigment und den oben aufgeführten mittel- oder/und grobkörnigen Partikeln werden erfindungsgemäße Testelemente erhalten, die zum photometrischen Nachweis im UV-Bereich, z.B. zum Nachweis von NADH bei 340-370 nm, sowohl in reflexionsphotometrischen Verfahren als auch in fluorimetrischen Verfahren geeignet sind.

Beim fluorimetrischen Nachweis von Analyten ist es nicht erforderlich, einen Reaktionsfilm zu verwenden, der Pigmente enthält. Es zeigt sich jedoch auch in diesem Fall, dass eine Optimierung von Messzeit und Signalintensität durch Schichtdicke, Reagenzkonzentration und Porosität des Film bestimmt werden kann.

Der Einschicht-Reaktionsfilm kann nach bekannten Methoden auf dem Träger aufgebracht werden. In der einfachsten Ausführungsform wird die für den Einschicht-Reaktionsfilm benötigte Beschichtungsmasse in der gewünschten Schichtdicke auf den Träger gegeben, z.B. durch Gießen, durch Aufstreichen mittels Bürsten, Pinsel oder Rakel oder durch Walzenauftrag oder durch eine Kombination dieser Verfahren, und getrocknet. Die Befestigung des Reaktionsfilms auf dem Träger kann ebenfalls nach bekannten Methoden, z.B. durch Adhäsionsfolien bzw. Klebebänder, erfolgen.

Neben Träger und Einschicht-Reaktionsfilm kann das Testelement noch weitere Komponenten enthalten. So kann eine Spreitauflage auf dem Reaktionsfilm vorhanden sein, welche die Probenflüssigkeit gleichmäßig und möglichst schnell dem Reaktionsfilm zuführt bzw. sie gegebenenfalls auf mehrere nebeneinander auf dem Träger vorhandene Reaktionsfilme gleichmäßig verteilt. Gegebenenfalls kommt der Spreitauflage darüber hinaus die Aufgabe zu, Überschüsse der Analytprobe von dem Testelement abzuleiten.

Das Testelement kann außerdem einen Auftragsbereich für Probenmaterial, z.B. einen Kapillarspalt bzw. einen Kapillarkanal, enthalten, welcher zum Flüssigkeitstransport vom Probenaufgabenbereich des Testelements zu einer Nachweiszone auf dem Einschicht-Reaktionsfilm dient. Daneben können Schutzabdeckungen, Abstandshalter, Adhäsionsschichten sowie Beobachtungs- und Messöffnungen vorhanden sein. Hinsichtlich des Aufbaus geeigneter Testelemente wird u.a. auf EP-A-0 995 994 bzw. WO 99/29429 verwiesen.

Das erfindungsgemäße Verfahren zum Nachweis eines Analyten umfasst das Inkontaktbringen der Probe mit einem erfindungsgemäßen Testelement und einen qualitativen Nachweis oder eine quantitative Bestimmung der Analytkonzentration in der Probe. Die quantitative Bestimmung erfolgt vorzugsweise durch optische Methoden, insbesondere durch reflexionsphotometrischen und fluorimetrischen Nachweis. Weiterhin ist bevorzugt, dass die quantitative Bestimmung innerhalb einer Reaktionsdauer von < 5 s, insbesondere von 1-4 s, erfolgt.

Zur Durchführung des Verfahrens wird die Probenflüssigkeit auf das Testelement aufgegeben, beispielsweise auf einer dem Reaktionsfilm abgewandten Seite einer Spreitauflage, wobei günstigerweise soviel Probenflüssigkeit aufgebracht wird, dass die durch die Spreitauflage gelangende Flüssigkeit den Reaktionsfilm vollständig sättigt. Als Probenflüssigkeit kommen insbesondere Körperflüssigkeiten, wie Blut, Plasma, Serum, Urin, Speichel etc., in Frage. Blut oder von Blut abgeleitete Flüssigkeiten, wie Plasma oder Serum sowie Urin, sind besonders bevorzugte Probenflüssigkeiten.

Durch die im Reaktionsfilm stattfindende Reaktion zwischen Testreagenz und Analyt entsteht ein mit der Konzentration des Analyten in der Probenflüssigkeit korrelierendes Messsignal. Bei diesem Messsignal handelt es sich vorzugsweise um eine Farbänderung, worunter Farbbildung, Farbverlust oder Farbumschlag verstanden wird. Diese Farbänderung wird visuell oder mit Hilfe eines Gerätes, vorzugsweise reflexionsphotometrisch, quantitativ ausgewertet werden, wobei hierzu entsprechende Detektoren benutzt werden können. Alternativ kann auch eine direkte Anzeige des Analytgehalts über die Software der Messvorrichtung bewerkstelligt werden. In einer weiteren bevorzugten Ausführungsform handelt es sich bei dem Signal um eine Fluoreszenz, die durch Bestrahlung des Testelements Licht mit einer Anregungswellenlänge und Detektion des aus der Probe abgestrahlten Lichts mit einer Emissionswellenlänge erfolgt.

Weiterhin soll die vorliegende Anmeldung durch die nachfolgenden Beispiele näher erläutert werden:

### Beispiel 1: Testelement zur reflexionsphotometrischen Bestimmung von Glucose mit PQQ-abhängiger Glucosedehydrogenase

Die Beschichtungsmasse wird aus folgenden Komponenten - als Reinsubstanz oder in Form von Stammlösungen - in einem Becherglas durch Rühren gemischt:

| | |
|---|---|
| Wasser | 80,50 g |
| Methylvinylether-Maleinsäure-Copolymer | 1,36 g |
| Natriumhydroxid | 0,49 g |
| Tetraethylammoniumchlorid | 0,68 g |
| N-Octanoyl-N-methyl-glucamid | 0,34 g |
| Natrium-N-methyl-N-oleoyl-taurat Fällungskieselsäure FK 320 DS (Fa. Degussa, | 0,03 g |
| mittlerer Durchmesser 5 µm) Titandioxid E 171 (Fa. Kronos, mittlerer | 6,03 g |
| Durchmesser 0,2 µm) | 14,44 g |
| Polyvinylpropionat-Dispersion (50 Gew.-% in Wasser) | 5,77 g |
| N,N-Bis-(2-hydroxyethyl)-4-nitroso-anilin-hydrochlorid | 0,30 g |
| 2,18-Phosphormolybdänsäure-hexanatriumsalz | 2,22 g |
| Pyrrolochinolinchinon | 0,0032 g |
| Calciumchlorid-2-hydrat | 0,05 g |
| Glucosedehydrogenase rec aus Acinetobacter calcoaceticus EC 1.1.99.17 | 0,26 g (=180 kU) |
| 1-Hexanol | 0,16 g |
| 1-Methoxy-2-propanol | 4,26 g |
| Kaliumhexacyanoferrat(III) | 0,01 g |

Die Gesamtmasse wird mit NaOH auf einen pH-Wert von 6,8 eingestellt und dann mit einem Rakelspalt von 30 µm Höhe auf eine 125 µm dicke Polycarbonatfolie aufgetragen und getrocknet.

Ein 5 mm breiter Streifen des so hergestellten Reaktionsfilms wird mit der Folienseite auf ein gestanztes doppelseitiges Klebeband auf der Trägerschicht passgenau aufgeklebt. Direkt am Reaktionsfilm angrenzend werden auf beiden Seiten doppelseitige Klebebänder als Abstandhalter auf den Träger aufgeklebt. Danach wird die Schutzfolie der beiden doppelseitigen Klebebänder abgezogen. Auf diesen Verbund wird ein 20 mm breiter Streifen eines gemäß EP-A-0 995 994 hergestellten Spreitvlieses aufgelegt und durch Anpressen verklebt. Es werden zwei einseitige Klebebänder als Abdeckungen so auf das Spreitvlies aufgeklebt, dass die Abstandhalter kann abgedeckt werden und wenigstens noch eine geringfügige Überlappung mit dem Reaktionsfilm stattfindet. Die auf diese Weise fertig gestellte Bandware wird in 6 mm breite Testelemente geschnitten, so dass das Messloch im Testelement mittig liegt.

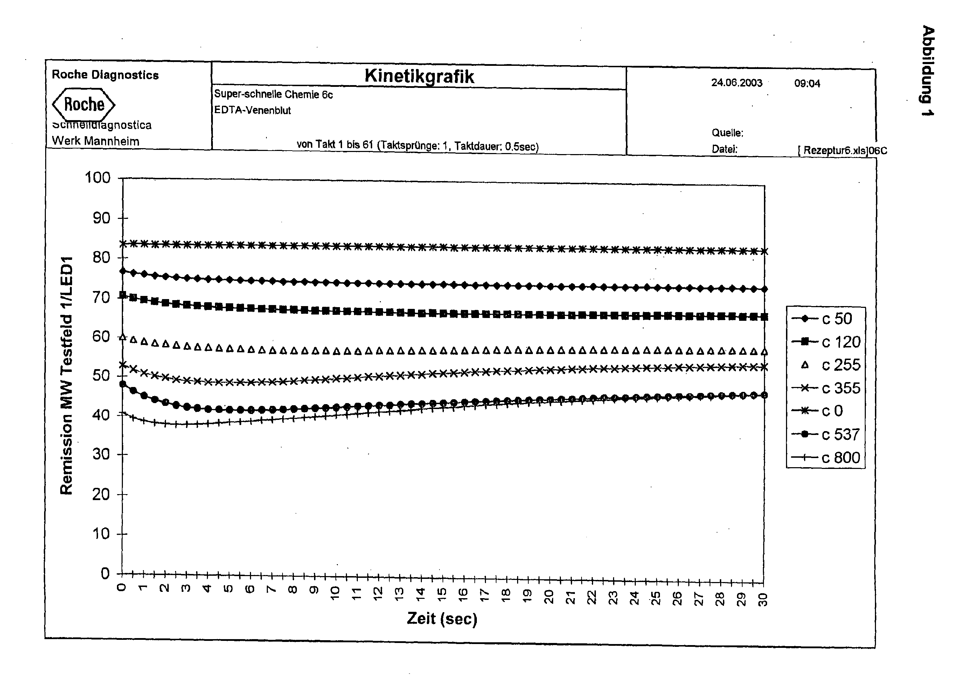

Die Testelemente werden mit verschiedenen Glucosekonzentrationen in EDTA-Venenblut an PC-gesteuerten Accu-Check® Active-Geräten vermessen. Die erhaltenen zeitlichen Verläufe der Farbentwicklung sind in Abb. 1 wiedergegeben. Wegen der eingesetzten Software sind die ersten 3 Messtakte in 0,5 s-Abstand in der Abbildung nicht enthalten.

Es wird gefunden, dass ein mit TiO₂ pigmentierter Einschichtfilm von ca. 7 µm Dicke (trocken) ein Messsignal von ca. 45 % Remission (absolut) für den Messbereich von 10-600 mg/dl Glucose aufweist. Die Reaktionszeit liegt bei etwa 1-2 s.

### Beispiel 2: Testelement zur reflexionsphotometrischen Bestimmung von Glucose mit GlucDOR

Nach der in Beispiel 1 beschriebenen Methode wird ein Testelement hergestellt, wobei anstelle von Glucosedehydrogenase GlucDOR (Wildtyp) verwendet wird. Das feinkörnige Pigment (Titandioxid, mittlerer Durchmesser 0,2 µm) wird mit mittel- oder grobkörnigen Partikeln wie folgt verdünnt:
(a) Mischen von Pigment und grobkörnigem Füllstoff, z.B. 14 g TiO₂ (0,2 µm Teilchengröße, enge Verteilung) + 6 g SiO₂ (5 µm, breite Verteilung)
(b) Mischen von Pigment und mittelkörnigem Füllstoff, z.B. 14 g TiO₂ (0,2 µm Teilchengröße) + 6 g TiO₂ (2-3 µm, mittlere Verteilung) oder BaSO₄ (1 µm, mittlere Verteilung)
(c) Mischen von Pigment und mittelkörnigem Füllstoff, z.B. 9 g TiO₂ (0,2 µm Teilchengröße) + 4,5 BaSO₄ (1 µm) + 4,5 g SiO₂ (5 µm)

Der Feststoffgehalt der Beschichtungsmasse wird dabei annähernd konstant gehalten.

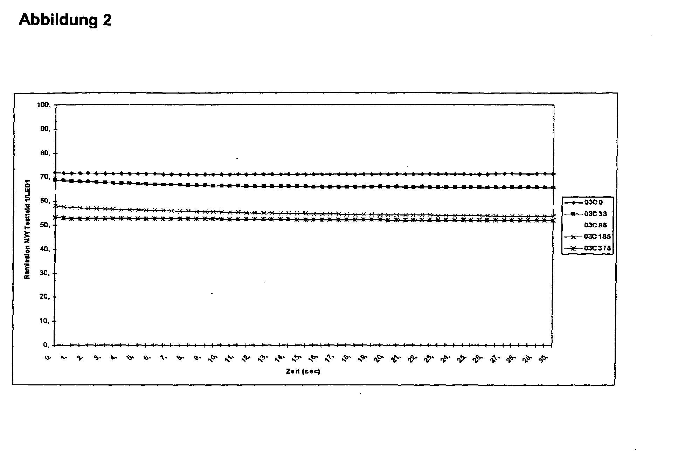

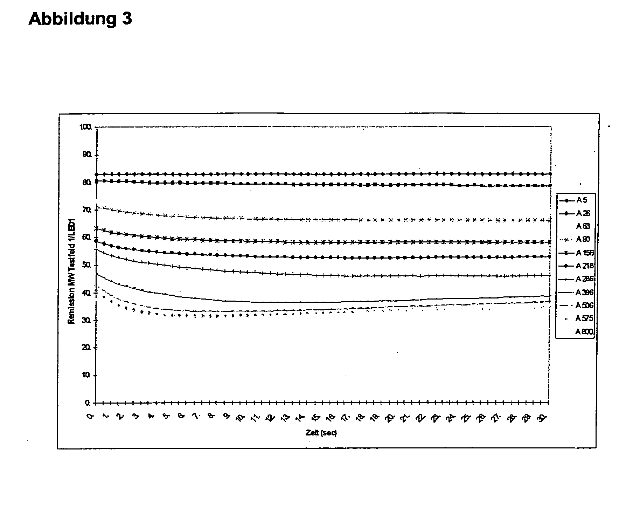

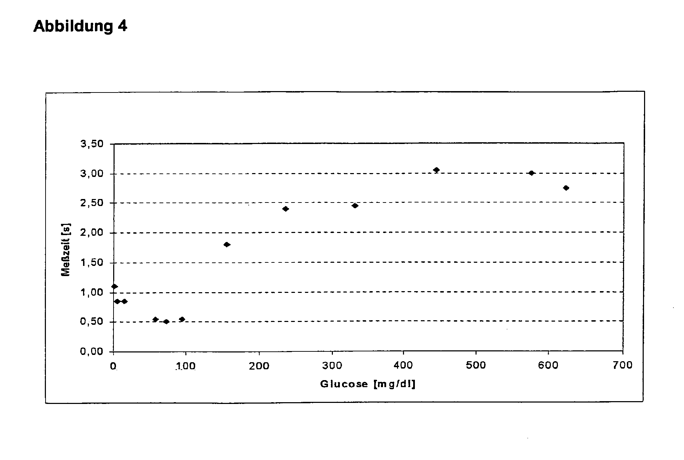

Die Messergebnisse für eine Bestimmung von Glucose in EDTA-Venenblut sind für Ansatz (a) in Abbildung 2, für Ansatz (b) in Abbildung 3 und für Ansatz (c) in Abbildung 4 gezeigt.

### Beispiel 3: Testelement zum fluorimetrischen Nachweis von Glucose durch Glucosedehydrogenase (GlucDH)

Es werden eine Polymerisations- und eine Imprägnierlösung aus folgenden Komponenten hergestellt:

| **Polymerisationslösung** | |
|---|---|
| GlucDH (50 % NaCl) | 27 U/mg 5,8 g |
| Puffer pH 7,0, FLUKA | 20,3 g |
| β-NAD⁺, freie Säure | 0,9 g |
| Puffer (FLUKA) pH 7 | 10,04 g |
| Ethanol | 8,3 g |
| N-Acryloyl-trishydroxymethyl-aminomethan | 2,1 g |
| Poly-(vinylsulfonsäure) Natriumsalz | 2,1 g |
| Initiator | 2,1 g |
| pH-Wert | 8,30 |
| pH, eingestellt mit HCl | 6,02 |

| **Imprägnierlösung** | |
|---|---|
| Benzophenon (5 %) | 2,00 g |
| Aceton | 4,00 g |
| Ethanol | 4,00 g |
| | **10,00 g** |

Eine Pokalonfolie (140 µm) wird mit Hilfe einer Rakelanlage mit der Imprägnierlösung beschichtet (10 µm Nassschicht) und anschließend in einem Trockenofen bei 50 °C für 15-20 min getrocknet.

Anschließend wird die Polymerisationslösung in einen Kunststoffuntersetzer gegeben und die vorimprägnierte Folie ausgelegt. Durch die Folien hindurch wird mit Hilfe einer Lampe (Fluotest Forte, Quecksilberdampflampe, kein Filter, Wellenlänge 280 nm - 360 nm) die Mischung auf die Folie aufpolymerisiert. Der Überstand wird mit Wasser abgewaschen und anschließend wird der aufpolymerisierte Film im Trockenschrank bei 50 °C für 15 min getrocknet.

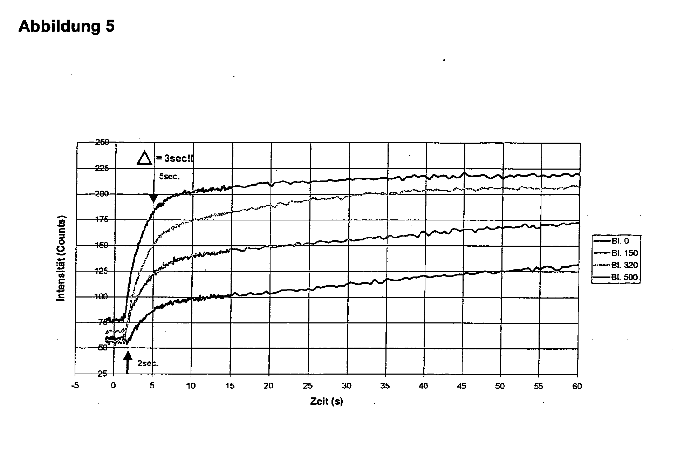

Abbildung 5 zeigt die zugehörige Blutmessung. Die anfänglichen Werte zeigen den Trockenwert des Testelements, die Benetzung findet etwa innerhalb von 2 s statt. Das Plateau wird bei ca. 5 s erreicht, so dass eine Messzeit von etwa 3 s ausreicht.

## Patentansprüche

1. Testelement zum optischen Nachweis eines Analyten, umfassend:
(a) einen zumindest teilweise optisch im wesentlichen transparenten Träger und
(b) einen Einschicht-Reaktionsfilm, enthaltend ein Testreagenz zur Bestimmung des Analyten mit einer Dicke ≤ 10 µm (trocken).

2. Testelement nach Anspruch 1,
**dadurch gekennzeichnet,**
**dass** der Träger eine Kunststofffolie ist.

3. Testelement nach Anspruch 1 oder 2,
**dadurch gekennzeichnet,**
**dass** das Testreagenz mindestens ein Enzym und mindestens ein nach enzymatischer Reaktion direkt oder indirekt nachweisbares Enzymsubstrat umfasst.

4. Testelement nach einem der Ansprüche 1 bis 3,
**dadurch gekennzeichnet,**
**dass** die Dicke des Reaktionsfilms im Bereich von 2-10 µm (trocken) ist.

5. Testelement nach einem der Ansprüche 1 bis 4,
**dadurch gekennzeichnet,**
**dass** die Konzentration des Testreagenz im Einschicht-Reaktionsfilm durch Optimierung der Intensität eines Messsignals eingestellt ist.

6. Testelement nach einem der Ansprüche 1 bis 5,
**dadurch gekennzeichnet,**
**dass** die Konzentration des Testreagenz im Einschicht-Reaktionsfilm derart ist, dass eine im wesentlichen gleichmäßige Reaktion mit dem Analyten über die Dicke des Testelements erfolgt.

7. Testelement nach einem der Ansprüche 1 bis 6
**dadurch gekennzeichnet,**
**dass** der Einschicht-Reaktionsfilm eine poröse Struktur aufweist.

8. Testelement nach Anspruch 7,
**dadurch gekennzeichnet,**
**dass** die Porengröße durch Optimierung der Intensität eines Messsignals eingestellt ist.

9. Testelement nach Anspruch 7 oder 8,
**dadurch gekennzeichnet,**
**dass** die Porengröße derart ist, dass ein Eindringen von Erythrozyten in den Film im wesentlichen ausgeschlossen ist.

10. Testelement nach einem der Ansprüche 1 bis 9,
**dadurch gekennzeichnet,**
**dass** der Einschicht-Reaktionsfilm feinkörnige Pigmentpartikel, beispielsweise Pigmentpartikel auf Basis von TiO₂ oder/und ZrO₂, enthält.

11. Testelement nach Anspruch 10,
**dadurch gekennzeichnet,**
**dass** der mittlere Durchmesser der feinkörnigen Pigmentpartikel ≤ 0,5 µm, insbesondere zwischen 0,1 µm und 0,3 µm ist.

12. Testelement nach einem der Ansprüche 1 bis 11,
**dadurch gekennzeichnet,**
**dass** der Einschicht-Reaktionsfilm
(i) mittel- oder/und grobkörnige Pigmentpartikel, beispielsweise Pigmentpartikel auf Basis von TiO₂ BaTiO₃, ZrO₂ ZrSiO₃ oder/und BaSO₄ oder/und
(ii) mittel- oder/und grobkörnige Partikel eines nichtreflektierenden Füllmaterials, beispielsweise SiO₂ oder/und eines Silikats oder/und eines wasserunlöslichen Kunststoffs, enthält.

13. Testelement nach Anspruch 12,
**dadurch gekennzeichnet,**
**dass** der mittlere Durchmesser der mittelkörnigen Partikel im Bereich zwischen 0,7 und ≤ 4 µm, insbesondere zwischen 1 und 3 µm, ist.

14. Testelement nach Anspruch 12 oder 13,
**dadurch gekennzeichnet,**
**dass** der mittlere Durchmesser der grobkörnigen Partikel im Bereich von ≥ 4 µm, insbesondere zwischen 4 und 8 µm, ist.

15. Testelement nach einem der Ansprüche 12 bis 14,
**dadurch gekennzeichnet,**
**dass** es ein Gemisch aus feinkörnigen Pigmentpartikeln und grobkörnigen Partikeln (i) oder/und (ii), insbesondere in einem Gewichtsverhältnis von 1 : 0,2-1 enthält.

16. Testelement nach einem der Ansprüche 12 bis 14,
**dadurch gekennzeichnet,**
**dass** es ein Gemisch aus feinkörnigen Pigmentpartikeln und mittelkörnigen Partikeln (i) oder/und (ii), insbesondere in einem Gewichtsverhältnis von 1 : 0,2-1 enthält.

17. Testelement nach einem der Ansprüche 12 bis 14,
**dadurch gekennzeichnet,**
**dass** es ein Gemisch aus feinkörnigen Pigmentpartikeln, grobkörnigen Partikeln (i) oder/und (ii) und mittelkörnigen Partikeln (i) oder/und (ii), insbesondere in einem Gewichtsverhältnis von 1 : 0,1 bis 0,9 : 0,1-0,9 enthält.

18. Testelement nach einem der Ansprüche 1 bis 17,
**dadurch gekennzeichnet,**
**dass** das Testreagenz in einer Polymermatrix vorliegt.

19. Testelement nach einem der Ansprüche 1 bis 18 zum reflexionsphotometrischen Nachweis eines Analyten.

20. Testelement nach einem der Ansprüche 1 bis 18 zum fluorimetrischen Nachweis eines Analyten.

21. Testelement nach einem der Ansprüche 1 bis 20 zum Nachweis im sichtbaren Bereich oder/und im UV-Bereich.

22. Testelement nach Anspruch 21 zum Nachweis von NADH.

23. Testelement nach einem der Ansprüche 1 bis 22,
**dadurch gekennzeichnet,**
**dass** es für eine Bestimmung des Analyten innerhalb einer Reaktionsdauer von < 5 s, insbesondere von 1-4 s, vorgesehen ist.

24. Testelement zum optischen Nachweis eines Analyten, umfassend:
(c) einen zumindest teilweise optisch im wesentlichen transparenten Träger und
(d) einen Einschicht-Reaktionsfilm, enthaltend ein Testreagenz zur Bestimmung des Analyten
**dadurch gekennzeichnet,**
**dass** es für eine Bestimmung des Analyten innerhalb einer Reaktionsdauer von < 5 s, insbesondere von 1-4 s, vorgesehen ist.

25. Testelement zum optischen Nachweis eines Analyten, umfassend:
(c) einen zumindest teilweise optisch im wesentlichen transparenten Träger und
(d) einen Einschicht-Reaktionsfilm, enthaltend ein Testreagenz zur Bestimmung des Analyten
**dadurch gekennzeichnet,**
**dass** Dicke und Porosität des Reaktionsfilms und Konzentration des Testreagenz derart eingestellt sind, dass eine Bestimmung des Analyten innerhalb der Reaktionsdauer von < 5 s, insbesondere von 1-4 s, bei einer für eine quantitative Bestimmung ausreichenden Signalintensität erfolgt.

26. Testelement nach einem der Ansprüche 1 bis 25 zur Bestimmung eines Analyten, ausgewählt aus Glucose, Lactat, Ketonkörpern und Harnstoff.

27. Testelement nach Anspruch 26 zur Bestimmung von Glucose im Blut.

28. Testelement nach Anspruch 27,
**dadurch gekennzeichnet,**
**dass** Testreagenz ein Enzymsystem umfasst, ausgewählt aus:
(i) Glucose-Dye-Oxidoreduktase oder
(ii) Glucose-Dehydrogenase.

29. Verfahren zum Nachweis eines Analyten in einer Probe, umfassend die Schritte:
(c) Inkontaktbringen der Probe mit einem Testelement nach einem der Ansprüche 1 bis 28 und
(d) quantitative Bestimmung der Analytkonzentration in der Probe.

30. Verfahren nach Anspruch 29,
**dadurch gekennzeichnet,**
**dass** die quantitative Bestimmung durch optische Methoden, insbesondere durch reflexionsphotometrischen oder fluorimetrischen Nachweis erfolgt.

31. Verfahren nach einem der Ansprüche 29 oder 30,
**dadurch gekennzeichnet**
**dass** die quantitative Bestimmung innerhalb einer Reaktionsdauer von < 5 s, insbesondere von 1-4 s, erfolgt.

32. Verfahren nach einem der Ansprüche 29 bis 31, zum Nachweis von Glucose.

33. Testvorrichtung zum optischen Nachweis eines Analyten in einer Probe,
**dadurch gekennzeichnet**
**dass** es ein Testelement nach einem der Ansprüche 1 bis 28 enthält.
